# EUROPEAN PATENT APPLICATION

(11) **EP 0 792 677 A1**
(43) Date of publication of application: **03.09.1997**
(21) Application number: 97200349.5
(22) Date of filing: 10.02.1997
(51) Int. Cl.: B01D 39/16

(54) **Leucocyte depleting filter-device, media and method of use**

(30) Priority: 28.02.1996 US 607089
(71) Applicant: Arbor Technologies, Inc., Ann Arbor, Michigan 48108 (US)
(72) Inventor: Haddock, Thomas F., Ann Arbor, Michigan 48104 (US); Monty, E. Vincent, Ann Arbor, Michigan 48103 (US)
(74) Representative: Wharton, Peter Robert

(57) **Abstract**

A filtering assembly (10) for removing leukocytes from a fluid is disclosed. The assembly (10) includes a housing (12) defining a chamber (14), an inlet (16) disposed in fluid communication with the chamber (14) for allowing a flow of fluid into the chamber (14), and an outlet (18) disposed in fluid communication with the chamber (14) for allowing a flow of fluid from the chamber (14). A biologically-active filter media (20) is disposed within the chamber (14) for removing leukocytes from the fluid passing through the assembly (10).

A filter media (20) for removing leukocytes from a fluid includes biologically-active moieties for removing leukocytes from the fluid is disclosed.

A method of removing leukocytes from a fluid by passing a fluid which contains leukocytes through a biologically-active filter media (20) for selectively and biochemically binding and retaining leukocytes to the filter media (20) from the fluid is also disclosed.

## Description

### TECHNICAL FIELD

The invention relates to an apparatus, media, and method for filtering blood, particularly an apparatus and method for removing leukocytes from blood.

### BACKGROUND OF THE INVENTION

Each year in the United States approximately ten million transfusions of blood or blood components take place. Blood is rarely administered as whole blood, but rather as individual blood components. The three predominant blood products produced from a unit of blood are the oxygen-carrying red cells, the clot-promoting platelets, and the noncellular plasma. Transfusions of packed red blood cells (PRBC or RBC) or platelet concentrates (PCs) contain, in addition to the specified cell types, large quantities of leukocytes (approximately 10⁸ - 10⁹ per unit). It has been estimated that as much as ninety percent of adverse transfusion reactions are caused by these donor leukocytes. Chief among these are the development by the recipient of antibodies to the Human Leukocyte Antigens (HLA sensitization).

This condition has many ramifications. The patient may become refractory to platelet transfusions whereby transfused platelets are destroyed by the recipient's immune system. This is a serious condition in patients requiring multiple transfusions, such as oncology patients. HLA sensitization may also give rise to non-hemolytic febrile transfusion reactions upon subsequent transfusions, making matching for organ transplants extremely difficult and cause failure of bone marrow transplants. Other adverse outcomes associated with the transfusion of donor leukocytes include graft-versus-host disease (GVHD), damage to red-cells or platelets in the stored unit due to the release of enzymes from the leukocytes as they break down with time, the production of inflammatory mediators during storage, immune suppression, and the transmission or reactivation of cytomegalovirus (CMV). CMV is a condition relatively harmless in healthy people, but potentially serious in patients with comprised immune systems. At the present time, only about twenty percent of homologous transfusions are filtered to remove leukocytes. However, due to the growing body of evidence indicating the undesirability of donor leukocytes, this number is expected to grow dramatically.

PRBCs and PCs usually contain 10⁸ to 10⁹ leukocytes per unit. Most transfusion recipients receive two or more units of PRBCs and pools of six to ten PCs at a time. Thus a patient may receive more than 10¹⁰ donor leukocytes with each transfusion. In addition, many patients today with diseases such as leukemia or liver disease receive numerous blood transfusions. Recent investigations have suggested that the total number of leukocytes transfused is the critical factor in preventing leukocyte-related complications. Febrile transfusion reactions may be prevented by reducing the transfused leukocyte load to a level of less than 5 X 10⁸ WBC, whereas preventing of HLA alloimmunization may require levels of less than 5 X 10⁶ WBC (Klein et al., 1992). Currently available filters may not consistently achieve sufficient reduction to prevent HLA immunization. The prevention of GVHD requires even greater levels of leukocyte reduction, which exceeds the capacity of current filters.

Leukocyte depletion of blood components can take place either at the bedside or in the blood center where the blood components are manufactured. While both procedures now take place, it is generally felt that leukocyte depletion at the time of manufacture is preferable, since it prevents the release of activation or breakdown products into the blood.

There is currently no technology that allows the removal of leukocytes from the whole blood (blood as it is drawn, before separation of individual components takes place), while retaining negligible amounts of red cells and platelets. When leukodepleted PRBC and PC are made from a single donation, each must be individually filtered. This necessitates one filter for the PRBC. After enough platelets have been pooled to constitute a therapeutic dose (usually collected from 6 to 10 whole-blood units), a separate filter for the platelets is required.

The use of two filters, with its associated complexity and expense, is necessary because current leukocyte-depletion technology will not permit the filtration of whole blood. Current leukocyte filters for PRBC retain platelets, which tend to adhere to surfaces. Leukocyte filters for PCs must be very specialized to permit the passage of the platelets. Hence they are significantly more expensive than those used for PRBC. A filter capable of filtering leukocytes from whole blood, hence allowing the production of leukodepleted PRBC and PCs prior to component manufacture, would increase efficiency and cut costs for the production of leukodepleted blood.

Current methods to reduce leukocytes from transfused blood rely on techniques relating to the physical characteristics of the surfaces of the filter media (e.g. surface energy and charge, as discussed in U.S.P.N. 4,923,620 and 4,925,572), and to the mechanical configuration of the media for simple sieving (e.g. effective pore size, density gradients, fiber denier, as discussed in U.S.P.N. 4,330,410). None of these devices have exploited the biological properties of blood cells.

Therefore, a filter and method for removing leukocytes from blood components by employing their specific biochemical binding sites would be desirable. This would permit stronger and more specific leukocyte binding forces, and hence allow the filtration of whole blood.

There are now no leukocyte filters on the market employing ligand binding techniques. The great majority of filters on the U.S. market are based on technologies that carefully control the surface energies, or other physical characteristics of the filter media. They are not based on specific ligand adhesion techniques. Much of the current technology rests on the control of the physical configurations of the nonwoven mats of fiber material, e.g. the fiber diameter and packing density. No leukocyte filters exist which are based on ligand adhesion techniques.

The commonly-used measure of merit for a leukocyte-reduction filter is its "Log Reduction Value" or "LRV," defined as the negative base 10 logarithm of the leukocyte reduction ratio produced by a single pass through the filter. Commercially available filters typically have LRVs of 2 to 3 (1:100 to 1:1000) and there are some claims of greater reduction values. LRVs less than 3 are sufficient to prevent febrile transfusion reactions, but not other, more serious, complications such as HLA immunization. This degree of leukocyte reduction may not be consistently achievable in actual clinical practice. Furthermore, currently available filters designed for PRBCs will not work for PCs, necessitating duplication of filtration devices in many instances. As more experience with the clinical use of leukocyte reduction filters has accumulated, many authorities have recognized a need for more efficient methods.

The leukocyte-reduction filter and method of the present invention permit greater levels of leukocyte reduction than are currently achievable. By employing biological mechanisms to specifically bind leukocytes, LRVs of 4 or greater may be reliably produced. This provides a consistent leukocyte reduction at clinically sufficient levels and, due to the filter's specificity, it can filter whole blood as well as blood components (PRBCs or PCs). Not only would this decrease costs by using one instead of two filters, but it will improve the quality of platelet concentrates by eliminating undesirable leukocyte activation and breakdown products. A product with higher levels of removal, as well as the possible filtration of whole blood, is a marked advance in transfusion technology.

### SUMMARY OF THE INVENTION AND ADVANTAGES

In accordance with the present invention, there is provided a filter assembly for removing leukocytes from a fluid. The filter assembly includes a housing having inlet means disposed in fluid communication with the chamber for allowing a flow of fluid into the chamber, outlet means disposed in fluid communication with the chamber for allowing a flow of fluid from the chamber and biologically-active filter means disposed within the chamber for removing leukocytes from the fluid passing through the assembly.

Also in accordance with the present invention, there is provided a filter media for removing leukocytes from a fluid which includes a biological moiety for removing leukocytes from the fluid.

Also in accordance with the present invention, there is provided a method of removing leukocytes from a fluid by passing a fluid which contains leukocytes through a biologically-active filter for selectively and biochemically binding and retaining leukocytes to the filter from the fluid.

### BRIEF DESCRIPTION OF THE DRAWINGS

Other advantages of the present invention will be readily appreciated as the same becomes better understood by reference to the following detailed description when considered in connection with the accompanying drawings wherein:
Figure 1 is a cross-sectional drawing of a typical filter design embodying the present invention;
Figure 2 is a graph illustrating the number of leukocytes per microliter separated from whole blood using magnetic beads coated with anti-CD62L versus beads coated with Mouse IgG as an indicator of the viability of the CD62L (L-selectin) over a twenty-four hour experimental interval;
Figure 3 illustrates the viability of CD62L expressed on neutrophils and lymphocytes; and
Figure 4 illustrates CD62L viability for the monocytes and eosinophils.

### DETAILED DESCRIPTION OF THE INVENTION

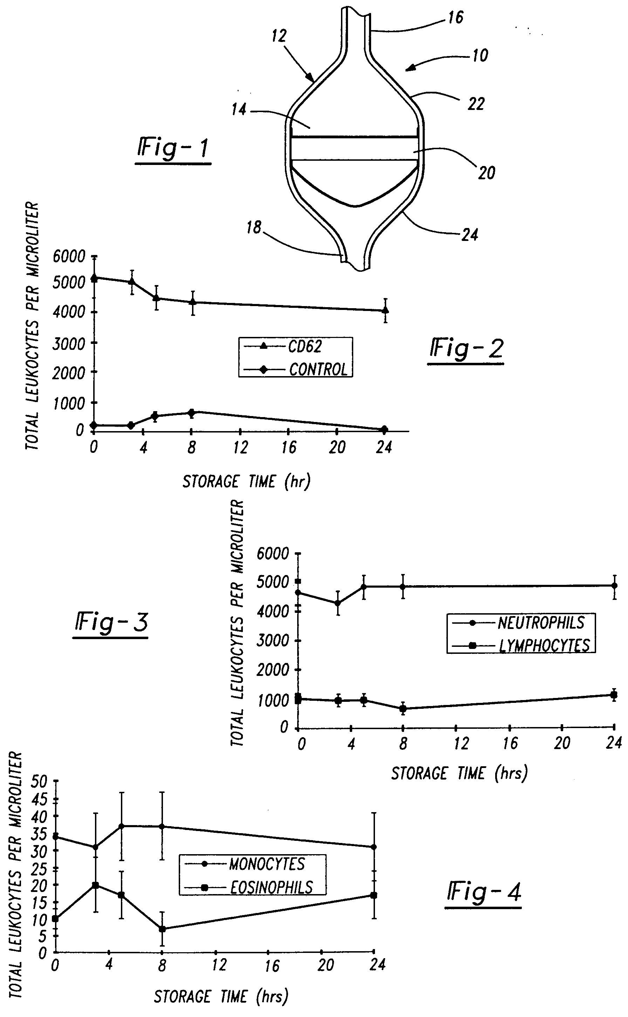

Referring to Figure 1, a filter assembly is generally shown and designated by the reference numeral 10. The filter assembly 10 is of a conventional filter design as is well known in the art. The filter assembly 10 includes a housing 12 which defines an inner chamber 14. An inlet 16 disposed in fluid communication with the chamber 14 allows for a flow of fluid to enter into the chamber 14. The filter assembly 10 further includes an outlet 18 disposed in fluid communication with the chamber 14 which allows the flow of fluid to exit from the chamber 14. The assembly 10 further includes a biologically-active filter 20 disposed within the chamber 14 for removing leukocytes from the fluid passing through the assembly 10.

More specifically, the housing 12 can be manufactured from suitable medical plastic materials, for example polypropylenes, polyolefins, polyesters, polyamides, polycarbonates, polystyrenes, styrenes, co-polymers, and fluoroplastics. This list is not meant to be exhaustive and can include other suitable materials without departing from the present invention.

The housing 12 is preferably made from two housing halves 22, 24, the two housing halves 22, 24 defining two walls 22, 24 of the housing 12.

The housing halves 22, 24 can be connected together by means well known in the art such as by gluing, sonic welding, or any other fusion-type bonding, but is not limited to these methods.

The inlet generally indicated at 16 is located proximate to a first end 13 of the housing 12, the inlet 16 being in fluid communication with the chamber 14 for allowing the flow of fluid into the chamber 14. The outlet, generally shown at 18, is proximate to a second end 15 of the housing 12 and in fluid communication with the chamber 14 for allowing a flow of fluid from the chamber 14 out of the housing 12.

A biologically-active filter 20 can be supported by the housing 12 and includes a surface layer of covalently-bonded or otherwise strongly retained (e.g., by dendrimers) ligands such as carbohydrates, lipids, proteins, and other moieties known to those skilled in the art, to which leukocytes in donor blood will bind specifically and efficiently, through biologic receptors. These ligands can be attached by polymeric chains extending from their attachment points on the surface of the polymers. For examples, carbohydrates can be disposed at a distance from the surface of the polymers. These polymers can be comprised of materials to which blood cells (including platelets) have low affinity. The density of the chains on the surface can serve to shield the underlying substrate from blood cells that may adhere to the substrate. The biologically-active filter 20 can be a mat, web, or solid material with the ligands, such as carbohydrates, with the ligands affixed thereto or can include an internal surface of the chamber 14 coated with the ligands. Two main criteria for ligand selection are that the ligand:
1) bond selectively and tightly to leukocytes, without significantly retaining red bloods cells or, ideally, retaining platelets,
2) can be strongly (e.g., covalently) bonded to an appropriate polymeric substrate without loss of activity.

Biologically-active is defined to include the interaction of biological molecules having specific affinity and specificity for each other, e.g., enzyme-substrate. That is, the ligands bind with specificity to other biological moieties or molecules in a manner such as that exhibited by enzyme-substrate binding. This would include specific means such as covalent binding, hydrogen binding and the like.

The ligands of the present invention can include mannose phosphate and fucoidan polysaccharides, two established carbohydrate ligands of L-selectin. This selection of candidate ligands can be based on established ligands for L-selectin (see below). These oligosaccharides and polysaccharides are available commercially, or can be synthesized from readily available precursors. Other suitable carbohydrate ligands can be polysaccharides and oligosaccharides including Lewis-x, Lewis-a, sialyl-Lewis x, sialyl-Lewis a, mannose-6-phosphate, polyphosphosmannose ester (PPME), fucoidan, and the glycolipid 3-O-sulfate-galactosyl ceramide. Additionally, synthesized sulfate derivatives of Lewis-a and Lewis-x can be used. In addition, the ligands could be another naturally-occurring or synthesized molecule.

Acceptable substrate media or solid support material and their surface treating chemicals for the filter means 20 of the present invention can include nylon, for its easy bonding characteristics, and polyester, as it is commonly used in blood-filtration as its surface can be easily derivatized. These two substrates can be treated, for example, with NaOH under a range of conditions to produce carboxylic acid and hydroxyl groups on the polyester, and carboxylic acid and amines on nylon. Cellulose, since it has many hydroxyls on the surface, and is easy to derivatize can also be a suitable material for the substrate of the biologically-active filter 20. Basic treatment of polyester and nylon substrates will produce surface structures on which carbohydrate ligands or polymeric attachment chains can be attached.

Platelet adhesion can be reduced by coating any of these substrates with, for example, acrylamide attachment sites (as discussed below) and these acrylamide sites would also be easily derivatized to attach the carbohydrate ligands. Nylon, polyester and cellulose acetate would still be likely candidates for substrate material, since they are readily processed to produce nonwoven fibrous mats which could be coated to produce the final filtration media, as noted below.

Nylon and polyester are both excellent filtration media with strong fibers but, more importantly, their surfaces can be hydrolyzed to provide free amines and hydroxyls respectively as well as free carboxylic acids. This hydrolysis breaks the bonds in the polymer chain and thereby weakens it structurally, but only on the fiber's surface.

Regenerated cellulose has hydroxyls readily available but is a weaker material. Cellulose acetate fibers are also weaker but can be readily hydrolyzed to produce free hydroxyls without significant chain scission.

Plastic fibers or sheets can be hydrolyzed in NaOH (various temperatures and times) as needed. First, appropriate hydrolysis conditions to obtain ample free amines (from nylon) as detected by Ninhydrin Reagent spray are obtained. Polyester hydrolysis requires harsher conditions and surface hydrolysis to free carboxylic acids is detected by the color change induced in a pH indicator dye. If required, acrylamides are attached to the free amines and hydroxyls on nylon, or on the free hydroxyls and carboxylic acid on polyester.

Nylon 6,6, poly (hexamethylene adipamide) is available from AIN Plastics, Inc., Southfield, Michigan as 1/16-inch sheets. Cerex TM filter media is available from RFiberweb Group at various basis weights appropriate for efficient blood filtration. Polyester, polyethylene terephthalate, sheets can be obtained from AIN plastics as type A, cloudy (i.e., crystalline) sheets of 0.010 inch thickness. Polyester filtration media can be obtained from Freudenberg, from Hollinsworth & Vose, or from Fiberweb Group, in various basis weights.

Membranes, i.e., surface filters, are not useful for filtering large volumes of blood because microaggregates in the blood easily plug the pores and shut down flow. The filters 20 are preferably filters with depth which are more open and which will not be so easily clogged. In addition, depth filters allow extended exposure (i.e., provide more surface area per unit volume) of the filtrate to the surfaces of the fiber media, as are necessary for obtaining good LRVs. The mechanism of separation needs to be specific adsorption, not sieving. Depth filters, also called filtration media, are porous mats of fibers. They have a nominal pore size rating but this is a very crude approximation. Basis weight, measured in ounces per square yard, is determined by the thickness of individual fibers, distance between fibers, and thickness of the mat; all these affect filtration efficiency and will be varied within a suitable range. Effective pore sizes can range from, for example, seven microns to 15-20 microns as red blood cells must pass through the filter. This range is provided for example purposes only and is not intended to be all inclusive as other suitable pore size ranges can be contemplated.

Materials which have chemically reactive functional groups for covalent binding of ligands are preferred. The substrate or solid support media must also be mechanically strong and available as porous webs suitable for blood filtration. Pure cellulose filter paper has an abundance of hydroxyl groups but is not very strong when wet. Nylon and polyester are both excellent filtration media with strong fibers but they have practically no reactive groups as supplied. However, the surfaces of nylon and polyester fibers can be hydrolyzed to provide free amines and hydroxyls respectively as well as free carboxylic acids. This hydrolysis breaks the bonds in the polymer chain and thereby weakens it structurally, but only on the fiber's surface.

Platelet adhesion to the substrate can be reduced by using, for example, acrylamide or acrylic acid attachment sites on the surface of the substrate material. Other inhibitors of platelet adhesion can include polymers that minimize platelet adhesion including poly (ethylene)/poly (propylene oxide)/poly (ethylene oxide) triblock copolymers (Pluronics) (Amiji et al., 1992). Recent work with graft polymerization of acrylamide on polyurethane has shown the number of platelets adhering to the polyacrylamide-grafted surfaces (graft density greater than 20 µg/cm²) was reduced by 95% compared to control surfaces (Fujimoto et al., 1993). Sections of the substrate not coated with carbohydrate-containing polymers can have many acrylamide attachment sites, and hence tend to repel platelets. The original surface can be completely coated, although it is unlikely that this would be necessary.

Once the underlying surface is treated to have a given acrylic acid density, the attachment procedure is very versatile and only minimally dependent on the type of substrate, allowing attachment of various types of block polymer chains. The concentration of monomers on the substrate surface to control the graft density can be altered, and hence the density of polymeric chains extending from the surface.

Acrylic acids are used as the attachment points, since acrylamides are water soluble and are easily worked. Acrylic acid chains can be grown to different lengths from the substrate surface. This allows variation of the effective surface density of carbohydrates, as well as to control the spectrum of cell sizes that may "push aside" the carbohydrate chains and contact the substrate surface. The latter effect is used to shield the uncoated substrate surface from platelets. There is likely an upper limit to this density above which cells of the sizes of platelets cannot reach the original surface. In this case, the adhesion of platelets is effectively blocked by the presence of the polymeric chains attached to the surface.

In addition, inserting various block polymer sections in the carbohydrate-ligand-containing polymers attached to the surface can be used. Low platelet adhesion has been observed on surfaces with attached polymers containing blocks such as poly(ethylene glycol) and poly(ethylene oxide)/poly(propylene oxide)/poly(ethylene oxide) triblock copolymers (Pluronics) (Amiji et al., 1992). These and other block polymers are used to alter the attached carbohydrate-ligand-containing polymers to control the activation and adhesion of platelets. By varying the proportions of various monomers in these chains between different types it is possible to achieve a carbohydrate density giving good leukocyte adhesion while incorporating sufficient sections of platelet-phobic block polymers to prevent excessive platelet adhesion. This allows control over the degree of binding of leukocytes and platelets individually and arrive at a mix where a high degree of leukocyte adhesion and a low degree of platelet adhesion is achieved.

The variables in this approach are:
1. carbohydrate selection to bind to biological receptors on leukocytes;
2. monomer or block polymer selections to resist platelet adhesion;
3. type and surface density of attachment sites; and
4. sequences of ligands and block polymers along with chains.

Of the various known leukocyte selectins, one known as L-selectin appears to be the foremost candidate for exploitation by a leukocyte filtration device. The distribution of L-selectin (also called CD62L, Leu-8, MEL-14) on blood leukocytes includes lymphocytes, neutrophils, monocytes and eosinophils where it is constituatively expressed. L-selectin mediated binding will occur under conditions of shear flow. Leukocyte rolling along the blood vessel wall, prior to firm attachment and extravasation involves L-selectin. The natural ligands of L-selectin appear to be sialyl-Lewis x (sLe^{x}) and sialyl-Lewis a (sLe^{a}) antigens. Data show that L-selectin is expressed on most blood leukocytes for up to twenty-four hours when stored in CPDA-1 (see below). Other leukocyte adhesion molecules with carbohydrate ligands include E-selectin and P-selectin which are not found on resting leukocytes, but are expressed by activated leukocytes. Both E-selectin and P-selectin have been shown to bind sLe^{x}, although with different affinities than L-selectin. sLe^{x} and other fucosylated lactosamines are also found on circulating neutrophils, monocytes, and some lymphocytes.

In addition to Lewis antigens, a number of carbohydrate structures may bind to L-selectin including mannose-6-phosphate, a yeast core polysaccharide PPME, sulfated polysaccharides such as fucoidan, and the glycolipid 3-O-sulfate-galactosyl ceramide. Modification of Lewis antigens may increase the affinity of E-selectin binding (Nelson et al., 1993). Addition of 8-methocycarbonyloctyl aglycone to N-acetyl glucosamine of sLe^{x} or sLe^{a} increases binding two-fold. Amino substitution for 2-N acetyl group of N-acetyl glucosamine (GlcNAc) of sLe^{a} increases affinity 36-fold. Both the sialic acid and fucose are required for activity, since their removal results in loss of E-selectin binding. Sulfated Le^{a} is also more potent ligand for E-selectin, having 15-fold greater affinity than sLe^{x} and 45-fold greater affinity than sLe^{a} (Yeun et al., 1984).

The literature suggests that receptors for carbohydrate ligands, including L-, E-, and P-selectin, are present on a broad spectrum of leukocytes. Less is known about binding of leukocytes to the specific carbohydrates we propose. Specifically, NK cells express sLe^{x} and bind to E-selectin (Pinola, 1994). Similarly, CD34+ stem cells express an L-selectin ligand (Oxley, 1994). Moore et al. (1992) showed that a subpopulation of CD4+, CD8+, and CD16+ lymphocytes memory cells bound P-selectin, which was sialidase sensitive. Peripheral blood dendritic cells appear to bind endothelial cells through E-selectin (Hill et al., 1994 and Srinivas, 1993). L-selectin is expressed by hematopoietic progenitor cells at various maturational stages (Kobayashi, 1994). Our data indicate that eosinophils and basophils express L-selectin, thus, the available data supports the hypothesis that a large proportion of circulating leukocytes have membrane receptors for carbohydrate ligands.

There are other possible approaches to binding leukocytes through biological mechanisms. One such approach is employing monoclonal antibodies (MAb) to specific leukocyte antigens, immobilized on a matrix, to bind leukocytes. In this case a broad range of MAbs are required to sufficiently retain the leukocytes. MAbs are very expensive as they must be produced by hybridoma technology. Oligosaccharides can be made much easier and less expensively by biochemical synthesis.

In an alternative embodiment of the present invention, lectins can be used to bind leukocytes. A lectin is a carbohydrate binding protein with a high degree of specificity. Using lectins to retain leukocytes by binding to sugars on their surfaces is the opposite technique from the above, where L-selectin on the leukocytes would be used to bind to a carbohydrate ligand on the filter material. In the former case, the receptor (lectin) is fixed to the filter, while in the latter the receptor (L-selectin) is intrinsic to the leukocyte. Lectins specific to leukocyte surface structures are derived from various plant and animal sources. However, proteins are not as durable as carbohydrates, and this is a factor in fastening them to the surfaces of polymers and having them stay viable there for a period of time. The preferred approach is to use carbohydrates to bond the L-selectin on the leukocyte surfaces.

The present invention can include at least one additional filter to perform normal filtering operations.

The present invention also includes a method of removing leukocytes from a fluid, such as blood, by passing the fluid containing the leukocytes through a biologically-active filter for selectively and biochemically binding and retaining the leukocytes to the filter from the fluid.

According to this method, a fluid containing leukocytes can be applied to a biologically-active filter media as discussed-above thereby specifically binding and removing the leukocytes from the fluid while allowing the remainder of the fluid to pass.

### EXAMPLES

Experiments demonstrating that active ligand technology can be effective in retaining leukocytes is presented below.

Materials: Plastic sheets AIN Plastics, Inc., Southfield, Michigan were cut to the size of microscope slides to use as models for the plastic material of porous filter webs. Of the many materials available as both fibrous media and as plastic sheets, nylon-6, 6 and polyester have potentially reactive functional groups that can be used for covalent immobilization.

Immobilization Methods: The surface of the slides was hydrolyzed in warm NaOH solution which produced free amine groups (from nylon, a polyamide) and free hydroxyls (from the polyester) as well as free carboxylic acids (from both). These functional groups are know to react with a variety of coupling compounds useful for immobilization. Ethylene glycol diglycidyl ether (EGDGE) were used since it is a water soluble di-epoxide known to react with hydroxyls on membranes and filters (Klein et al., 1994). The surfaces of the slides were reacted with one of the two epoxides of EGDGE. Then the epoxide of EGDGE was reacted with the hydroxyls on either of two ligands: Fucoidan or D-mannose-6-phosphate. Three variations of ligand and concentrations were tried, as described below. Also, two variations of immobilizing the saccharides in one step were attempted by combining them with EGDGE. The sixth variation was a control (NaOH hydrolysis, but no EGDGE and no ligand). These immobilizations were done on only the right hand side of the slide; the left hand side (EGDGE but no ligand) served as a internal control.

Test Results: 10 ml of PRBC (CPDA-1) were allowed to flow over the treated surface of the slides for 12 minutes. The slides were then washed in buffer, fixed, stained with Wright-Giemsa, and examined visually and microscopically. This demonstrated enhanced binding and aggregation of leukocytes by EGDGE-coated slides (both nylon and polyester) treated with 4% fucoidan, or 8% mannose phosphate, or 4% mannose phosphate plus 50% organic wetting agent, compared to the controls. There was no leukocyte binding to control surfaces lacking carbohydrate ligands.

Two treatments of 10% fucoidan plus 1% EGDGE (one on EGDGE-coated slides, the other on hydrolyzed slides without EGDGE pretreatment) gave mixed results; but not as good as the first three. This result was interpreted to mean that there exists an optimum amount of covalent immobilization and that too high a concentration of coupling agent will destroy the binding site of the ligand. Generally, the polyester series gave better binding than the nylon in spite of the higher reactivity of amines over hydroxyls. Apparently, the hydroxyls reacted sufficiently and non-ligand-specific binding may be better for polyester.

These results demonstrate the utility of the porous filter of the present invention for filtering leukocytes using similar chemistry to immobilize ligands on nylon, polyester and other materials.

For the work to be of clinical value, carbohydrate binding receptors must be expressed on a large percentage of leukocytes over the time between when the blood is drawn and when leukodepletion takes place. Ideally, a whole blood filter could be used shortly after the donor blood is drawn, but in normal practice the process may be delayed up to several hours. In order to better understand the degree of L-selectin expression as a function of time we performed a series of experiments.

A study by Wikman et al. (1994) of stored granulocyte concentrates showed that L-selectin is expressed at basal levels for at least four hours, but is markedly reduced at twenty-four hours. (p. 168. Wikman). Inversely, MAC-1 (CD11b/CD18) is not expressed on fresh and four hours old granulocytes, but is present at twenty-four hours. In this study, donors were pretreated with prednisone to induce demargination, and hydroxyethyl starch was used as a sedimenting agent. These treatments themselves may down-regulate L-selectin expression (Stibenz, 1994). Data indicate that L-selectin expression is maintained for at least eight hours at room temperature and twenty-four hours at 4°C during storage of CPDA-1 whole blood. Since whole blood should not be held for more than eight hours before the manufacture of platelet concentrate, alterations of leukocyte receptor expression beyond this time may not be significant. Eight hours would allow ample time for filtration of whole blood at a collection facility.

Since L-selectin is a labile leukocyte adhesion receptor, studies were performed to analyze the stability and distribution of L-selectin on blood leukocytes during storage under normal blood collection conditions. Monoclonal anti-CD62L (L-selectin) or control mouse Abs (of the same isotype) was coupled to magnetic beads, as a model of leukocyte-surface interactions. 200µl of CPDA-1 whole blood stored up to twenty-four hours in Fenwall PL 146 bags was incubated with 4x10⁶ beads for thirty minutes. The beads were separated, and total and differential leukocyte counts were performed on the bead and supernatant samples.

Figure 2 illustrates the number of leukocytes per microliter separated from whole blood using magnetic beads coated with anti-CD62L versus beads coated with mouse IgG. L-selectin expression was significantly greater than the control and was stable over twenty-four hours.

Figures 3 and 4 illustrate binding of anti-CD-62L coated beads to the major leukocyte subpopulations proportionate to their numbers in whole blood over twenty-four hours. Due to small numbers of monocytes, and eosinphils, experimental errors are relatively large in these graphs. Basophils, while not plotted, were also adherent to anti-CD62L coated beads but not to control beads. In this experiment cells had less interaction opportunities with the surface of the beads than would be present in a filter device. Thus the number of leukocytes removed by this technique was significantly less than would occur in a filter device based on the same principles, and this may have limited our detection accuracy. Microscopic examination of supernatent samples after bead separation revealed that a significant proportion of the leukocytes not removed by magnetic separation had one to three beads attached. This may indicate that there are lower levels of L-selectin expression in some types of leukocytes. Even with these limitations our data indicate that a large proportion of leukocytes can be successfully removed from whole blood through a specific ligand to L-selectin.

The absolute level of L-selectin expression was basically stable under these storage conditions for over twenty-four hours. Neutrophils, lymphocytes, monocytes, eosinophils, and basophils bound anti-CD62L coated beads proportionately to their numbers in whole blood. Of note, samples incubated with anti-CD62L contained aggregates of leukocytes in which one or more leukocytes that had no attached beads were adherent to a leukocyte with attached beads. No such aggregates, and significantly fewer total aggregates, were seen in control samples. This suggests that leukocytes bound through L-selectin may express other adhesion molecules leading to secondary interactions. This is consistent with the results of Stockl, et al (1993).

Throughout this application various publications are referenced by citation or number. Full citations for the publication are listed below. The disclosure of these publications in their entireties are hereby incorporated by reference into this application in order to more fully describe the state of the art to which this invention pertains.

The invention has been described in an illustrative manner, and it is to be understood the terminology used is intended to be in the nature of description rather than of limitation.

Obviously, many modifications and variations of the present invention are possible in light of the above teachings. Therefore, it is to be understood that within the scope of the appended claims, reference numerals are merely for convenience and are not to be in any way limiting, the invention may be practiced otherwise than as specifically described.

### REFERENCES CITED

Amiji and Park, "Prevention of protein adsorption and platelet adhesion on surfaces by PEO/PPO/PEO triblock copolymers" Biomaterials, v. 13, n. 10, pp. 682-02 (1992).
Fujimoto et al., "Polyurethane surface modification by graph polymerization of acrylamide for reduced protein adsorption and platelet adhesion" Biomaterials, v. 14, n.6, pp. 442-8 (1993)
Hill et al., "Differential function of dendritic cells isolated from blood and lymph nodes" Immunology, v. 83, n. 2, pp. 295-301 (1994).
Klein et al., "Leukocyte-reduced blood component therapy" In McArthur (ed) Hematology (1992).
Klein et al., "Chitosan modified sulphonated poly (ethersulfone) as a support for affinity separations" Journal Membrane Science, v. 95, pp. 199-204 (1994).
Kobayashi et al., "Expression of adhesion molecules on human hematopoietic progenitor cells at different maturation stages" Stem Cells, v. 12, n.3, pp. 316-21 (1994).
Moore and Thompson "P-selectin (CD62) binds to subpopulations of human memory T lymphocytes and natural killer cells" Biochem Biophys Res Commun, v. 186, n.1, pp. 173-81 (1992)
Nelson et al., "Higher-affinity oligosaccharide ligands for E-selectin" J Clin Invest. v. 91, pp. 1157-1166 (1993)
Oxley and Sackstein et al., "Detection of an L-selectin ligand on a hematopoietic progenitor cell line" Blood, v. 84, n. 10, pp. 3299-306 (1994).
Pinola et al., "Characterization of the E-selectin ligand on NK cells" J Immunol, v. 152, n. 7, pp. 3586-94 (1994).
Srinivas et al., "E-selecting involvement in vitro adhesion of blood dendritic cells to human umbilical cord endothelial cells" Scand. J. Immunol, v. 38, n. 3, pp. 273-8 (1993).
Stibenz and Buhrer, "Down-regulation of L-selectin surface expression by various leukocyte isolation procedures" Scand. J. Immunol., v. 39. n.1, pp. 59-63 (1994).
Stockl et al., "Monoclonal antibodies to the carbohydrate structure Lewis^{x} stimulate the adhesive activity of leukocyte integrin CD11b/CD18 (CR3, Mac-1, αₘβ₂ on human granulocytes" J. of Leukocyte Biology, v. 53, pp. 541-549 (1993).
Wikman et al., "Altered expression of adhesion molecules (L-selectin and Mac-1) on granulocytes during storage" Transfusion, v. 34, n. 2, pp. 167-171 (1994).
Yeun, C-T, et al., "Sulfated blood group lewis^{a}. A superior oligosaccharide ligand for human E-selectin." J Bio Chem., v. 269, pp. 1595-1598 (1994).

## Claims

1. A filtering assembly (10) for removing leukocytes from a fluid, said assembly (10) comprising:
a housing (12) defining a chamber (14);
inlet means (16) disposed in fluid communication with said chamber (14) for allowing a flow of fluid into said chamber (14);
outlet means (18) disposed in fluid communication with said chamber (14) for allowing a flow of fluid from said chamber (14); and
biologically-active filter means (20) disposed within said chamber (14) for removing leukocytes from the fluid passing through said assembly (10).

2. A filter assembly (10) as set forth in claim 1, wherein said biologically-active filter means (20) includes ligands attached to a solid support means which specifically and biochemically bind and retain leukocytes and support said biologically-active filter means.

3. A filter assembly (10) as set forth in claim 2, wherein said ligands are carbohydrates which selectively bind to leukocytes.

4. A filter assembly (10) as set forth in claim 3, wherein said carbohydrates are Lewis antigens.

5. A filter assembly (10) as set forth in claim 3, wherein said carbohydrates bind to compounds selected from the group consisting essentially of L-selectin, E-selectin, and P-selectin.

6. A filter assembly (10) as set forth in claim 2, wherein said support means further includes a fibrous media.

7. A filter assembly (10) as set forth in claim 6, wherein said fibrous media is selected from the group consisting essentially of thermoplastics and cellulose.

8. A filter assembly (10) as set forth in claim 7, wherein said thermoplastic is selected from the group consisting essentially of nylon and polyester.

9. A filter assembly (10) as set forth in claim 1 further including inhibiting means for inhibiting platelet adhesion within said filter assembly (10).

10. A filter assembly (10) as set forth in claim 9, wherein said inhibiting means includes a surface treatment disposed on said support means.

11. A filter assembly (10) as set forth in claim 10, wherein said surface treatment includes acrylic acid.

12. A filter assembly (10) as set forth in claim 10, wherein the surface treatment includes acrylamide.

13. A filter assembly (10) as set forth in claim 9, wherein said surface treatment further includes polymers selected from the group consisting essentially of polyethylene glycol and polyethylene and polyethylene oxide/polypropylene oxide/polyethylene oxide triblock copolymers.

14. A method of removing leukocytes from a fluid by passing a fluid which contains leukocytes, through a biologically-active filter for selectively and biochemically binding and retaining leukocytes to the filter from the fluid.

15. A method as set forth in claim 14, wherein said passing step is further defined as passing the fluid through ligands bound to a solid support which specifically and biochemically bind and retain leukocytes.

16. A method as set forth in claim 15, wherein the ligands are carbohydrates.

17. A method as set forth in claim 16, wherein the carbohydrates are Lewis antigens.

18. A method as set forth in claim 16, wherein the carbohydrates bind to compounds selected from the group consisting essentially of L-selectin, E-selectin, and P-selectin.

19. A method as set forth in claim 14, including the step of binding the biologically-active filter to the support for retaining and supporting said biologically-active filter.

20. A method as set forth in claim 14, including the step of inhibiting platelet adhesion.

21. A method as set forth in claim 20, wherein said inhibiting step further includes disposing a surface treatment on the support.

22. A method as set forth in claim 21, wherein the surface treatment includes acrylic acid.

23. A method as set forth in claim 21, wherein the surface treatment includes acrylamide.

24. A filter media (20) for removing leukocytes from a fluid, said media (20) comprising:
biologically-active filter means (20) for removing leukocytes from the fluid.

25. A filter media (20) as set forth in claim 24, wherein said biologically-active filter means (20) includes ligands attached to a solid support means which specifically and biochemically bind and retain leukocytes and support said biologically-active filter means.

26. A filter media (20) as set forth in claim 25, wherein said ligands are carbohydrates which selectively bind to leukoctyes.

27. A filter media (20) as set forth in claim 26, wherein said carbohydrates are Lewis antigens.

28. A filter media (20) as set forth in claim 26, wherein said carbohydrates bind to compounds selected from the group consisting essentially of L-selectin, E-selectin, and P-selectin.

29. A filter media (20) as set forth in claim 25, wherein said support means further includes a fibrous media. 5

30. A filter media (20) as set forth in claim 29, wherein said fibrous media is selected from the group consisting essentially of thermoplastics and cellulose.

31. A filter media (20) as set forth in claim 30, wherein said thermoplastic is selected from the group consisting essentially of nylon and polyester.

32. A filter media (20) as set forth in claim 24 further including inhibiting means for inhibiting platelet adhesion within said filter assembly (10).

33. A filter media (20) as set forth in claim 32, wherein said inhibiting means includes a surface treatment disposed on said support means.

34. A filter media (20) as set forth in claim 33, wherein said surface treatment includes acrylic acid.

35. A filter media (20) as set forth in claim 33, wherein said surface treatment includes acrylamide.

36. A filter media (20) as set forth in claim 32, wherein said surface treatment further includes polymers selected from the group consisting essentially of polyethylene glycol and polyethylene an polyethylene oxide/polypropylene oxide/polyethylene oxide triblock copolymers.
